# FASCICULE DE BREVET EUROPEEN

(11) **EP 1 242 047 B1**
(45) Date de publication et mention de la délivrance du brevet: **22.09.2004**
(21) Numéro de dépôt: 00990848.4
(22) Date de dépôt: 29.12.2000
(51) Int. Cl.: A61K 9/107, A61K 9/48, A61K 47/22, A61K 31/215

(54) **FORMULATIONS GALENIQUES DU FENOFIBRATE ET LEUR PROCEDE D'OBTENTION**
FENOFIBRATHALTIGE GALENISCHE ARZNEIZUSAMMENSETZUNGEN UND VERFAHREN ZU IHRER HERSTELLUNG
FENOFIBRATE GALENIC FORMULATIONS AND METHOD FOR OBTAINING SAME

(30) Priorité: 31.12.1999 FR 9916807
(43) Date de publication de la demande: 25.09.2002
(73) Titulaire: FOURNIER INDUSTRIE ET SANTE, 21300 Chenôve (FR)
(72) Inventeur: LEMUT, Josiane, F-21000 Dijon (FR); BLOUQUIN, Pascale, F-21240 Talant (FR); REGINAULT, Philippe, F-21121 Fontaine-les-Dijon (FR)
(74) Mandataire: Hubert, Philippe
(86) Numéro de dépôt international: PCT/FR2000/003738
(87) Numéro de publication internationale: WO 2001/049262

(56) Documents cités:
- EP-A- 0 724 877
- WO-A-98/30205
- WO-A-99/12528
- WO-A-99/29300

## Description

La présente invention concerne de nouvelles formulations galéniques du fénofibrate administrables par voie orale, leur procédé d'obtention ainsi que les médicaments fabriqués à partir de ces formulations.

Le fénofibrate (DCI) est un principe actif de médicament connu depuis de nombreuses années en raison de son efficacité pour diminuer les taux de triglycérides et de cholestérol dans le sang. De ce fait, le fénofibrate est largement prescrit dans de nombreux pays quand il est nécessaire de diminuer le risque athérogène.

On sait également que. pour obtenir un effet hypocholestérolémiant satisfaisant, il est souhaitable de maintenir un taux circulant d'acide fénofibrique (qui est le métabolite actif du fénofibrate) de l'ordre de 6 à 10 mg/l. Un tel taux est notamment obtenu avec une dose unitaire de fénofibrate de 300 mg en gélule (cf. Drugs 40 (2) p 260-290 (1990)).

On sait aussi qu'il existe des variations importantes des taux circulants en fonction des pathologies observées chez les patients. D'une façon générale pour tous les médicaments, il est préférable de maintenir un taux circulant du métabolite actif nécessaire pour obtenir l'effet thérapeutique recherché tout en faisant absorber au patient une quantité minimale de principe actif. C'est pourquoi, on recherche des formulations présentant la plus haute biodisponibilité possible afin d'optimiser la posologie et de limiter d'éventuels effets secondaires du principe actif.

Enfin, on sait que la biodisponibilité des principes actifs (administrés par voie orale) peut varier si le médicament est absorbé à jeun, pendant les repas ou après les repas (voir par exemple Int. Journal of Clinical Pharmacology and Biopharmacy 16 p 570-574 (1978), Europ. Journal of Clinical Pharmacology 17 p 459-463 (1980), Drug Metabolism and Disposition 16 p 302-309 (1988)).

Compte tenu de ces éléments, on comprend que la formulation galénique d'un principe actif absorbé par voie orale présente une grande importance pour obtenir l'effet thérapeutique dans des conditions optimales.

Le fénofibrate a été commercialisé à l'origine sous forme de gélules dosées à 100 mg de principe actif, avec une posologie de 3 gélules par jour, puis sous forme de gélules dosées à 300 mg de principe actif, prescrites à raison d'une gélule par jour. Les études mentionnées précédemment font état, après administration d'une gélule dosée à 300 mg à des volontaires sains, d'une biodisponibilité de l'ordre de 30 %, avec un taux circulant d'acide fénofibrique maximum d'environ 6 à 9 mg/l et une aire sous courbe de 145 à 170 mg/l.h.

Une autre formulation divulguée dans le document EP 330 532, et commercialisée en France sous la marque LIPANTHYL 200M, résulte d'un procédé consistant à comicroniser le fénofibrate avec un composé tensioactif solide de façon à obtenir un mélange intime et finement divisé des deux produits. Ce type de formulation permet de réduire la posologie à 200 mg/jour en une seule prise pour obtenir des concentrations plasmatiques d'acide fénofibrique très proches de celles obtenues avec une prise de 300 mg de fénofibrate non comicronisé (Journal International de Médecine (1991) n° 206 p 48-50). Cette formulation correspond ainsi à une amélioration de la biodisponibilité de l'ordre de 30 %, par rapport à la formulation d'origine.

Un autre type de formulation a été proposé dans le document FR 2 494 112. Il s'agit ici de microgranules dans lesquelles un noyau neutre est enrobé de fénofibrate micronisé puis recouvert d'une couche de protection. La posologie préconisée, dans ce cas, est de 250 mg/jour, ce qui correspond à une biodisponibilité intermédiaire par rapport aux formulations précédentes.

Plus récemment, on a proposé dans le document FR 2 758 459 une composition sous forme de granulés ou de comprimés dans lesquels le fénofibrate, sous forme micronisée, est associé à un polymère hydrophile (notamment la polyvinylpyrrolidone) et éventuellement à un tensioactif. Les résultats obtenus avec un granulé conforme à cette formulation contenant un tensioactif, montrent une dissolution plus rapide du fénofibrate dans des tests de laboratoire. Une étude pharmacocinétique comparative montre également une biodisponibilité améliorée, notamment en considérant les paramètres de la concentration plasmatique en acide fénofibrique et de l'aire sous courbe.

On connaît également, par le document EP 757 911 un procédé de préparation d'une formulation de fénofibrate consistant à réaliser une solution du principe actif dans l'éther monoéthylique du diéthylèneglycol et à conditionner cette solution dans des capsules molles. Selon les résultats mentionnés dans ce document, l'administration de 100 mg/jour de fénofibrate permet d'obtenir les concentrations plasmatiques d'acide fénofibrique requises pour assurer l'efficacité du médicament. Il se pose toutefois le problème résultant de l'administration en quantité relativement importante du solvant par voie orale. En effet, selon les formulations décrites, la prise de 100 mg de fénofibrate correspond à la prise simultanée de 1500 mg d'un éther du diéthylèneglycol. Or, le fénofibrate étant un hypolipidémiant, le médicament est destiné à être prescrit sur des périodes prolongées, à raison, par exemple, de 100 mg /jour si la biodisponibilité de cette formulation a été doublée par rapport à celle de la composition décrite dans le document EP 330 532. De ce fait, l'utilisation de cette formulation reviendrait à faire absorber quotidiennement et régulièrement 1,5 g d'un éther du diéthylèneglycol dont les effets biologiques ne sont pas totalement neutres (cf. Food Cosmet. Toxicol. (1968) 6 (6) p689-705 ; Arzneim . Forsch (1978) 28(9) p 1571-1579 ; Occup. Hyg. (1996), 2 (1-6, Proceedings of the Int. Symposium on Health Hazards of glycol Ethers, 1994), 131-151).

On connaît également, selon 5^{ème} Congrès Intern. Technol. Pharm. Vol. 3 (1989) p 190-199, une formulation se présentant sous la forme d'une solution de fénofibrate dans le diméthylisosorbide en mélange avec un agent dispersant. Selon les essais mentionnés, on obtient essentiellement une biodisponibilité qui est beaucoup moins dépendante de la présence d'aliments dans le milieu gastrique, ce qui constitue déjà un progrès par rapport à la forme classique en gélules pour lesquelles la biodisponibilité serait variable de 26 % à jeun à 89 % après repas.

Une formulation proche de la précédente est proposée dans le document EP 904 781, qui préconise de préparer un co-fondu de fénofibrate et d'un dispersant solide tel que la croscarmellose ou la polyvinylpyrolidone.

Il est par aileurs connu, dans l'art de la galénique, qu'il est possible d'obtenir des mélanges intimes entre une phase huileuse lipophile habituellement insoluble dans l'eau et une phase aqueuse ou hydrophile, qui peuvent prendre la forme d'une dispersion de type eau-dans-huile ou huile-dans-eau, et qui, selon la taille des particules dispersées, sont qualifiées d'émulsions ou de microémulsions. Les microémulsions généralement caractérisées par une dimension des particules dispersées inférieure à 100 nm peuvent être obtenues par réduction des tensions superficielles au moyen d'agents tensioactifs et présentent l'avantage, par rapport aux émulsions, de former des compositions limpides et stables. De plus, ces microémulsions sont généralement préparées à partir d'un pré-concentré anhydre par simple mélange avec de l'eau ou avec le milieu gastrique, pratiquement sans apport d'énergie, en conduisant à une dispersion parfaite du principe actif (Journal Dispersion Science and Technology 6 (3) p 317-337 (1985) ou Progress in Surface and Membrane Science 12 p 405-477).

De ce fait, les microémulsions ont été mises à profit en thérapeutique dans le but de favoriser les échanges entre les phases lipidiques et les phases hydrophiles des milieux biologiques et obtenir ainsi une amélioration du passage des médicaments à travers les membranes des cellules.

Une formulation de fénofibrate sous forme de préconcentré de microémulsion a été décrite dans le document WO 99/29300.

Cette formulation comprend une phase lipophile dans laquelle le fénofibrate est solubilisé, une phase hydrophile et un système émulsifiant.

Les formulations décrites dans ce document antérieur comprennent toutes, à l'exception de la formulation de l'exemple 11, un composant hydrophile additionnel, tel qu'en particulier l'éthanol et/ou le propylène glycol, qui permet d'assurer une bonne solubilisation du fénofibrate dans la phase huileuse.

D'une façon générale, l'adjonction d'éthanol et/ou de propylène glycol dans une formulation huileuse facilite la dissolution du principe actif dans la phase lipophile et permet ainsi d'éviter les phénomènes de cristallisation du principe actif. Cependant, l'utilisation d'un tel composé hydrophile est incompatible avec la formulation d'un médicament sous forme de gélules scellées ou de capsules molles à base de gélatine, et limite donc l'intérêt d'une telle formulation.

Il a par ailleurs été constaté par les présents inventeurs que des formulations à base de fénofibrate uniquement constituées d'huile et d'un système émulsifiant telles que celle décrite à l'exemple 11 du document précité ne présentent généralement pas une stabilité entièrement satisfaisante, du point de vue de leur utilisation comme médicament, l'apparition de cristaux de fénofibrate pouvant être observée.

Il existe donc un besoin, non satisfait à ce jour, de disposer d'une formulation pharmaceutique à base de fénofibrate sous forme d'un préconcentré capable de former spontanément une microémulsion huile-dans-eau au contact d'un milieu aqueux, qui présente une très haute biodisponibilité du principe actif, et qui soit stable dans le temps, sans nécessiter l'utilisation d'un composant hydrophile additionnel limitant son utilisation pour la préparation de gélules scellées ou de capsules molles.

Il a été découvert, et ceci constitue le fondement de la présente invention, que l'acétate de vitamine E permet de stabiliser de façon durable dans le temps un pré-concentré à base de fénofibrate dont la phase lipophile est de préférence constituée d'une huile à base de glycérol ou de propylèneglycol estérifié, sans adjonction d'un quelconque composant hydrophile additionnel, et tout en conservant une très haute biodisponibilité du fénofibrate.

Ainsi, selon un premier aspect, la présente invention a pour objet une composition pharmaceutique pour l'administration par voie orale du fénofibrate sous forme d'un préconcentré capable de former spontanément une microémulsion huile-dans-eau au contact d'un milieu aqueux, comprenant :
- une phase lipophile comprenant de préférence une huile à base de glycérol ou de propylène glycol, de préférence encore totalement estérifié par des acides gras saturés à chaîne moyenne ;
- un système émulsifiant comprenant :
   . un tensioactif lipophile, de préférence à base de glycérol ou de propylèneglycol partiellement estérifié par des acides gras saturés à chaîne moyenne ;
   . un co-tensioactif hydrophile ;
caractérisée en ce qu'elle comprend en outre de l'acétate de vitamine E en une quantité suffisante pour stabiliser ledit préconcentré, sans adjonction d'un composant hydrophile additionnel.

Cette composition pharmaceutique est particulièrement avantageuse, dans la mesure où étant exempte de composant hydrophile elle permet la réalisation d'un médicament sous forme de gélules scellées ou de capsules molles à base de fénofibrate, présentant une stabilité prolongée et une excellente biodisponibilité. L'expression "composant hydrophile" s'entend. dans le cadre de la présente description, comme couvrant tout composant hydrophile autre que l'eau.

Par ailleurs, cette composition pharmaceutique permet de former spontanément sans apport d'énergie une microémulsion en présence d'une phase aqueuse ou en milieu gastrique présentant des gouttelettes dispersées de taille granulométrique généralement comprise entre 10 et 50 nm.

Cette composition est particulièrement stable lors du stockage à température ambiante, aucun phénomène de précipitation ou d'apparition de cristaux n'ayant été observé sur une durée prolongée.

Sur le plan pharmacocinétique, cette composition pharmaceutique est particulièrement remarquable en ce qu'elle permet de limiter les variations interindividuelles, et de réduire l'effet lié au repas tout en augmentant la biodisponibilité de façon importante, par rapport aux formulations connues obtenues par voie sèche.

Selon une caractéristique particulière de l'invention, l'acétate de vitamine E est présent, au sein de la composition pharmaceutique en une quantité telle que la dose journalière de vitamine E administrée soit d'environ au moins 100 Unités Internationales (UI), permettant une protection des lipoprotéines contre l'oxydation.

Alternativement, une partie de la vitamine E administrée peut provenir du système émulsifiant, lorsque celui-ci comprend, en tant que co-tensioactif, de la vitamine E TPGS (Succinate de l'alpha-D-Tocophérol et du polyéthylène glycol).

Des compositions pharmaceutiques particulièrement préférées dans le cadre de la présente invention contiennent, en quantités pondérales relatives :
a) 5 à 20 %, et de préférence 6 à 9 % de fénofibrate ;
b₁) 0 à 69 % et de préférence 0 à 40 % d'huile ;
b₂) 5 à 76 % et de préférence 5 à 50 % d'acétate de vitamine E ;
c₁) 3 à 69 % et de préférence 12 à 45 % de tensioactif ;
c₂) 3 à 69 % et de préférence 12 à 45 % de co-tensioactif.

L'huile entrant dans la composition de la phase lipophile est avantageusement choisie parmi des triglycérides d'acides gras saturés à chaîne moyenne, notamment des triglycérides d'acides gras saturés en C₈-C₁₂ et préférentiellement les acides gras saturés en C₈-C₁₀. Par acides gras saturés à chaîne moyenne, on comprend les acides gras saturés en C₈-C₁₂, c'est à dire les acides caprylique, caprique et laurique. Parmi les composés commercialisés convenant pour réaliser l'invention, on peut citer des produits vendus sous la dénomination CAPTEX par la société ABITEC, notamment les produits références CAPTEX 300, CAPTEX 350 et CAPTEX 355 qui sont essentiellement des triesters du glycérol avec des mélanges d'acides caprylique, caprique ou laurique. Parmi les produits de la même famille, habituellement obtenus à partir de l'huile de coprah, on peut utiliser également le MIGLYOL 812, commercialisé par la société HULS qui est un triglycéride des acides caprylique et caprique. La phase huileuse peut également comprendre des diesters du propylène glycol avec des acides gras en C₈-C₁₂, tels que par exemple un produit commercialisé sous la dénomination CAPTEX 200 par la société ABITEC et qui est un ester des acides caprylique et caprique avec le propylène glycol.

La phase lipophile peut être exempte d'huile, lorsque l'acétate de vitamine E, lui-même lipophile, est utilisée en quantité importante, par exemple supérieure à 25% en poids, rapporté au poids de la composition.

La composition selon l'invention comprend également, parmi ses constituants essentiels, un système émulsifiant généralement constitué d'un tensioactif lipophile associé à un co-tensioactif hydrophile.

Le tensioactif lipophile est de préférence un tensioactif non ionique dont la Balance Hydrophile-Lipophile (HLB) est inférieure à 12. Parmi les composés répondant à ces caractéristiques, on préfère des produits résultant de l'estérification partielle du glycérol ou du propylène glycol avec des acides gras saturés en C₈-C₁₀ (acides caprylique et caprique). A titre d'exemple on peut utiliser les produits commercialisés sous les références CAPMUL MCM, CAPMUL MCM C8, CAPMUL MCM C10 par la société ABITEC et qui sont des esters partiels du glycérol avec des quantités variables d'acide caprylique et d'acide caprique. On peut également utiliser le composé CAPTEX 200E6 (commercialisé par ABITEC) qui est un ester des acides caprylique ou caprique avec le propylène glycol soumis à une réaction avec l'oxyde d'éthylène.

Le co-tensioactif présent dans la formulation est à prédominance hydrophile, avec une HLB supérieure à 12. Parmi les composés susceptibles d'être utilisés dans le cadre de l'invention, on peut citer les esters de sorbitol et d'acides gras. copolymérisés avec l'oxyde d'éthylène. dénommés généralement polysorbates. Parmi les différents produits de cette catégorie, on préfère le polysorbate 80, commercialisé par exemple sous les références TWEEN 80 par la société ICI ou MONTANOX 80 par la société SEPPIC, qui est un monooléate de sorbitol copolymérisé avec environ 20 moles d'oxyde d'éthylène et qui présente une HLB de 15 environ. En tant que co-tensioactif, on peut également utiliser des produits de la réaction entre l'oxyde d'éthylène et les huiles de ricin naturelle ou hydrogénée, comme par exemple les produits commercialisés par la société BASF sous les références CREMOPHOR EL (obtenu par réaction de 35 moles d'oxyde d'éthylène avec environ 1 mole d'huile de ricin et qui présente une HLB d'environ 12 à 14) ou CREMOPHOR RH40 (obtenu par réaction de 40 moles d'oxyde d'éthylène avec environ 1 mole d'huile de ricin hydrogénée et qui présente une HLB d'environ 14 à 16).

Il est à noter que dans le cadre de la présente invention, il est possible d'utiliser dans le système émulsifiant le composé connu sous la dénomination TPGS (D-alpha-Tocophérylpolyéthylèneglycol 1000 succinate) qui présente un caractère amphiphile, avec une HLB comprise entre 15 et 19 .

L'utilisation de ce composé dans les compositions conformes à la présente invention est particulièrement avantageuse dans la mesure où ce composé constitue une source additionnelle de vitamine E qui permet une bonne protection des lipoprotéines contre l'oxydation à partir d'une dose journalière délivrée d'environ 100 Unités Internationales (UI).

La formulation peut éventuellement comprendre des additifs couramment utilisés en petite quantité, tels que par exemple des arômes ou des colorants.

Les constituants principaux (c'est à dire le principe actif, l'huile, l'acétate de vitamine E. le tensioactif et le co-tensioactif) doivent être présents en respectant des proportions relatives qui permettent d'obtenir une phase homogène susceptible de donner une microémulsion après dilution dans une phase aqueuse. De façon avantageuse, les proportions préférées selon l'invention permettent d'obtenir, à partir du préconcentré, une microémulsion pouvant inclure un fort pourcentage d'eau en présence d'une phase aqueuse, soit par exemple après une dilution jusqu'à 1/2500 voire après une dilution à l'infini. De plus les proportions des différents composants seront avantageusement choisies de telle sorte à permettre la délivrance quotidienne d'une dose de vitamine E d'environ au moins 100 UI, suffisante pour assurer une protection des lipoprotéines contre l'oxydation.

L'acétate de vitamine E ayant un effet solvant du principe actif doit être présent en quantité suffisante pour que la solution soit stable et éviter tout risque de cristallisation du fénotibrate. Si l'acétate de vitamine E est en quantité importante, c'est à dire qu'il représente par exemple plus de 25 % du poids de la formulation, il est possible de supprimer l'huile de la formule, la solubilisation du fénofibrate étant alors assurée par l'acétate de vitamine E.

Les constituants ayant un effet émulsifiant (tensioactif et co-tensioactif) doivent être présents en quantité suffisante pour que le préconcentré forme, après dilution avec une phase aqueuse. une microémulsion stable.

Ainsi, sur la base de ces principes, on a déterminé que :
- le principe actif doit être en solution dans la formulation huileuse et en quantité inférieure à la limite de saturation, soit, de façon pratique, inférieure à environ 20 % en poids rapportée au poids de la formulation et de préférence de l'ordre de 6 à 9% ;
- la quantité de phase lipophile (qui comprend l'huile et l'acétate de vitamine E) et la quantité d'agents émulsifiants (c'est à dire le tensioactif et le co-tensioactif) doivent être dans un rapport pondéral compris entre 1/15 et 5/1 et. préférentiellement entre 1/5 et 2/1;
- l'huile et l'acétate de vitamine E constituant la phase lipophile doivent être présents dans un rapport pondéral de l'ordre de 0 à 15/1 et préférentiellement de l'ordre de 0 à 10/1;
- les quantités de tensioactif et de co-tensioactif doivent être choisies dans un rapport pondéral compris entre 1/80 et 4/1 et préférentiellement 1/12 et 2/1.

Selon l'une des formules préférées de l'invention, les quantités pondérales de la phase huileuse et des émulsifiants sont environ du simple au double, avec pour la phase huileuse une quantité d'huile et une quantité d'acétate de vitamine E similaire et, pour les émulsifiants, une quantité de co-tensioactif sensiblement double de la quantité de tensioactif, le principe actif en solution dans le mélange représentant environ 6 à 9 % du poids de la préparation finale.

Selon une seconde formule préférée de l'invention, le rapport du poids de la phase huileuse au poids de la phase émulsifiante est de 2/3, avec pour la phase huileuse, une quantité d'acétate de vitamine E environ double de celle de l'huile et pour les émulsifiants des quantités similaires de tensioactif et de co-tensioactif, le principe actif représentant environ 7 % du préconcentré.

De telles formulations permettent d'obtenir des solutions particulièrement stables dans le temps, dans des conditions normales de conservation, et aptes à générer spontanément une microémulsion lorsqu'elles sont mélangées à une phase aqueuse, tout en comprenant une dose de vitamine E assurant avantageusement une protection des lipoprotéines contre l'oxydation.

Selon un second aspect, la présente invention a pour objet un médicament fabriqué à partir de la formulation préconcentrée décrite ci-dessus.

De façon pratique, le préconcentré sous forme de solution sera conditionné de préférence dans des gélules scellées ou dans des capsules molles en gélatine solubles dans le milieu gastrique de façon à libérer le préconcentré dans l'estomac et former la microémulsion en présence du suc gastrique.

Selon un autre mode de réalisation, le préconcentré peut être dilué extemporanément dans une boisson (eau, jus de fruit,...) pour former une microémulsion buvable. Cette forme peut être préférée par des patients qui ont des difficultés pour avaler des gélules ou des capsules molles et qui souhaitent un médicament buvable.

Les exemples suivants ont pour but d'illustrer l'invention et ne doivent pas être considérés comme limitatifs, notamment en ce qui concerne la nature et la quantité des différents excipients.

De même, le mode opératoire de fabrication décrit pour les exemples peut être modifié et un protocole différent par l'ordre d'addition des constituants ou les temps d'agitation peut être mis en oeuvre, en particulier si les appareillages sont plus ou moins performants.

### Exemples 1 à 4 : Mise en évidence de l'effet de l'acétate de vitamine E sur la solubilisation du fénofibrate dans l'huile.

On a évalué la solubilité du fénofibrate dans différentes formulations de phase huileuse ou de préconcentrés, en présence ou non d'acétate de vitamine E et les résultats suivants ont été obtenus :

| EXEMPLES | | Solubilité du fénofibrate en mg / g de mélange | |
|---|---|---|---|
| 1 | Captex 200 Huile | 130 mg / g | |
| | Captex 200/ vit E acétate 9/1 | 140 mg / g | |
| | Captex 200 / vit E acétate 3/7 | 170 mg / g | |
| | | | |
| 2 | Miglyol 812N Huile | 100 mg / g | |
| | Miglyol 812N/ vit E acétate 9/1 | 120 mg / g | |
| | Miglyol 812N / vit E acétate 3/7 | 145 mg / g | |
| | | | |
| 3 | (Capmul MCM / Tween 80 1/1) / Captex 200 50/50 | | 108 mg / g |
| | (Capmul MCM/ Tween 80 1/1) / (vit E acétate / Captex 200 3/7) 50/50 | | 123 mg / g |
| | | | |
| 4 | (Capmul MCM/ Tween 80 1/1) / (vit E acétate / Captex 200 3/7) 60/40 | | 110 mg / g |
| | (Capmul MCM/ Tween 80 1/1) / (vit E acétate / Captex 200 3/7) 50/50 | | 123 mg / g |
| (toutes les proportions sont exprimées en valeurs pondérales). | | | |

Les exemples 1 et 2 montrent clairement que l'incorporation d'acétate de vitamine E augmente sensiblement la solubilité du fénofibrate dans l'huile.

L'exemple 3 montre que l'incorporation de vitamine E acétate conduit également à une augmentation de la solubilité du fénofibrate en présence d'un système émulsifiant.

L'exemple 4 montre qu'une augmentation de la proportion du couple (acétate de vit E/Captex 200) dans la formulation comprenant les émulsifiants, améliore la solubilité du fénofibrate. On peut remarquer également, en comparant les exemples 3 et 4, que l'on obtient une même solubilité (108 et 110 mg/g) avec moins de phase huileuse en présence de vitamine E acétate.

### Exemples 5 et 6

On a évalué la stabilité de formulations préconcentrées à base d'huile et d'un système émulsifiant, en présence ou non d'acétate de vitamine E et les résultats suivants ont été obtenus :

| Exemple 5 | Taux de fénofibrate | Composition huile | Stabilité ambiante |
|---|---|---|---|
| 56% tensioactifs 37% huile | 7% | 37% Captex 355 | 7 mois puis cristaux |
| 56% tensioactifs 37 % huile | 7% | 26% Captex 355 + 11% vit E acétate | toujours stable à 18 mois |
| | | | |

| Exemple 6 | Taux de fénofibrate | Composition huile | Stabilité ambiante |
|---|---|---|---|
| 55% tensioactifs 37% huile | 8% | 37 % Captex 200 | 8 mois puis cristaux |
| 55% tensioactifs 37 % huile | 8% | 26 % Captex 200 + 11% vit E acétate | toujours stable à 18 mois |

Ces exemples montrent clairement que l'acétate de vitamine E permet d'améliorer sensiblement la stabilité du préconcentré à température ambiante.

### Exemple 7 : Préparation d'un médicament selon l'invention, sous forme de capsules molles

Dans un réacteur en verre, avec agitation de type impeller, on prépare un mélange de 434 g de polysorbate 80 (TWEEN 80, HLB ± 15) et 217 g d'un ester partiel du glycérol avec les acides caprylique et caprique (CAPMUL MCM, HLB ± 5 à 6). On mélange les deux produits pendant 15 mn, puis on ajoute 139,5 g d'acétate de vitamine E. Lorsque le mélange présente un aspect homogène, on ajoute 139.5 g d'un ester du glycérol avec les acides caprylique et caprique (CAPTEX 355). L'agitation est maintenue jusqu'à obtention d'une phase homogène puis on ajoute progressivement à température ambiante, 70 g de fénofibrate en poudre fine. La durée totale du mélange est au moins de 3 à 5 heures de façon à obtenir une dissolution parfaite. La solution huileuse ainsi obtenue est conditionnée en capsules molles contenant chacune 1 g de solution, soit 70 mg de fénofibrate.
Selon cette formulation, le rapport des quantités pondérales de tensioactif et de co-tensioactif est de 1/2, celui de l'acétate de vitamine E et de l'huile est de 1/1 et celui de la phase lipophile et des tensioactifs est de 3/7, la dose en vitamine E permettant d'assurer une dose journalière minimum de 280 UI administrée en deux prises ).

### Exemples 8 à 22 : Exemples de formulations conformes à l'invention

En opérant de façon analogue à l'exemple 7, on a préparé les formulations 8 à 22, dont les compositions qualitatives et quantitatives sont mentionnées dans le tableau 1 (les quantités sont exprimées en pourcentage pondéral et sont ramenées pour la vitamine E en nombre d'UI pour 1 g).
Dans ce tableau, les différents constituants, indiqués à titre non limitatifs sont les suivants :
- CAPMUL MCM est un ester partiel du glycérol avec les acides caprylique et caprique. Ce tensioactif présente une HLB de 5,5 à 6
- CAPMUL MCM C8 est un ester partiel du glycérol avec l'acide caprylique. Ce tensioactif a une HLB de l'ordre de 3 à 4.
- TWEEN 80 est un polysorbate 80, plus précisément un monoléate de sorbitol copolymérisé avec environ 20 moles d'oxyde d'éthylène. Ce co-tensioactif présente une HLB voisine de 15.
- Crémophor RH40 est le produit de la réaction de l'huile de ricin hydrogénée avec environ 45 moles d'oxyde d'éthylène. Ce co-tensioactif présente une HLB de environ 14 à 16.
- Crémophor EL est le produit de la réaction de l'huile de ricin avec environ 35 moles d'oxyde d'éthylène. Ce co-tensioactif présente une HLB voisine de 12 à 14.
- Vitamine E TPGS (ou TPGS) est un ester mixte de l'acide succinique avec la vitamine E et le polyéthylèneglycol 1000.
- Ac Vit E est l'acétate de la vitamine E
- CAPTEX 200 est un diester du propylène glycol avec les acides caprylique et caprique
- CAPTEX 355 est un triester du glycérol avec les acides caprylique (environ 57 %) et caprique (environ 40 %).

Les formulations de fénofibrate selon l'invention ont fait l'objet d'essais afin de déterminer la biodisponibilité du fénofibrate. Les essais ont été menés parallèlement avec des formulations sèches de fénofibrate, connues comme étant celles qui, actuellement, présentent les biodisponibilités les plus favorables. Les essais ont été conduits sur des rats mâles Sprague Dawley non à jeun auxquels on a administré par voie orale certaines des compositions selon l'invention. A titre de comparaison, on a également inclus dans le test des granulés (Féno Comp) obtenus selon l'enseignement du document de FR 2 758 459 et qui correspondent, selon ce document, à la formulation solide conduisant à la meilleure biodisponibilité, ainsi qu'une formulation (LIP) de fénofibrate comicronisé avec du laurvlsulfate de sodium telle que commercialisée en France sous la marque LIPANTHYL 200M. Les doses administrées correspondent à une administration d'environ 150 mg/kg de principe actif.
La cinétique a été établie pour chacun des essais en réalisant 10 prélèvements sanguins répartis sur 72 heures. La concentration en acide fénofibrique dans les sérums a été mesurée par HPLC (chromatographie en phase liquide) selon les méthodes analytiques connues. Les valeurs obtenues ont permis de déterminer les valeurs des divers paramètres considérés habituellement comme représentatifs de la biodisponibilité du principe actif :
a) l'aire sous courbe normalisée et extrapolée (AUC), exprimée en µg.h/ml
b) le temps de résidence moyen (TRM) exprimé en heures
c) la concentration maximale (C max.) atteinte dans le sérum (exprimée en µg/ml)
d) le temps au bout duquel la concentration maximale (T max.) est obtenue (exprimée en heures)

Les résultats issus de l'étude pharmacocinétique sont présentés dans le tableau II (Féno. Comp. est le premier exemple comparatif, LIP est le second exemple comparatif).

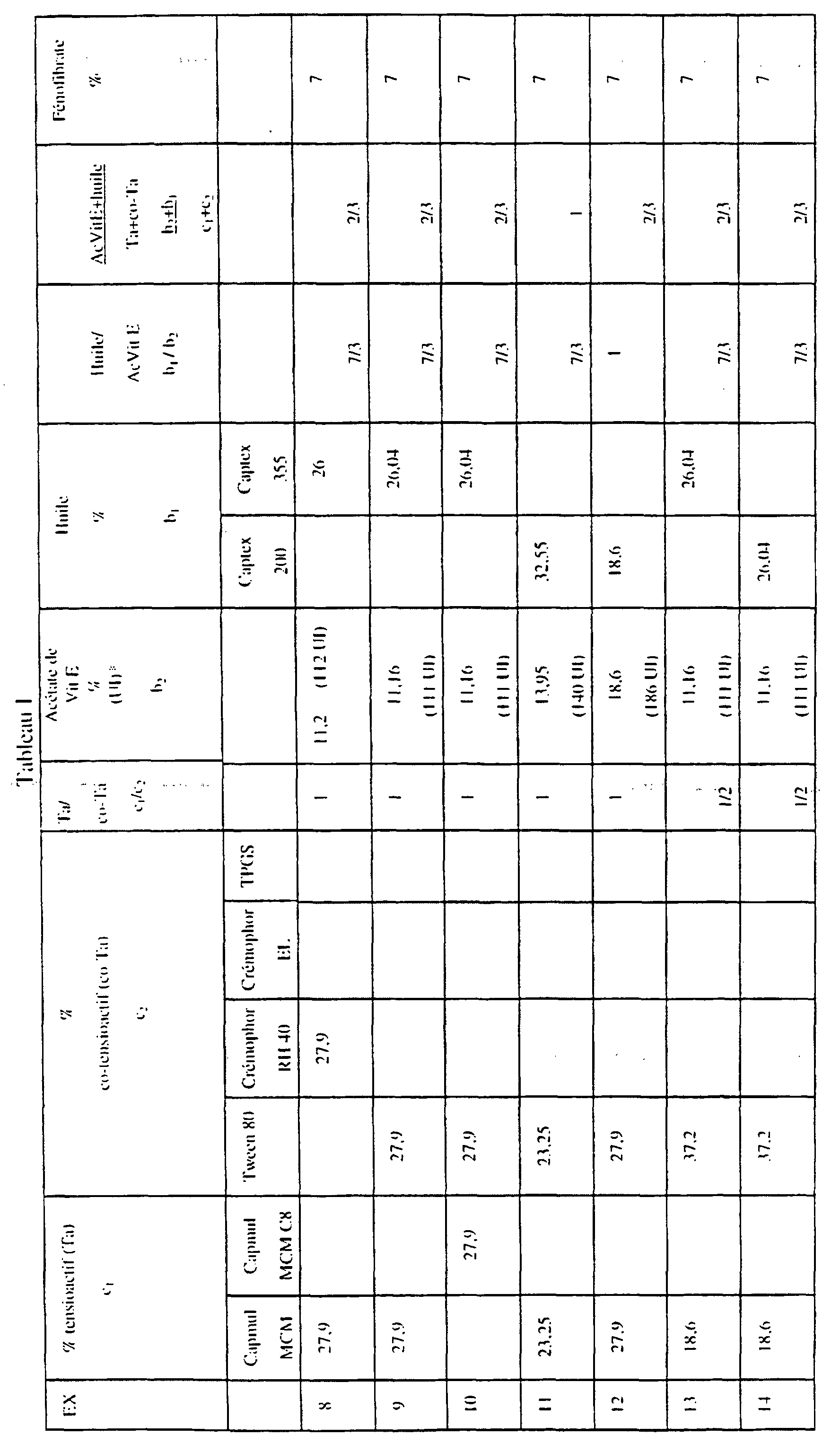

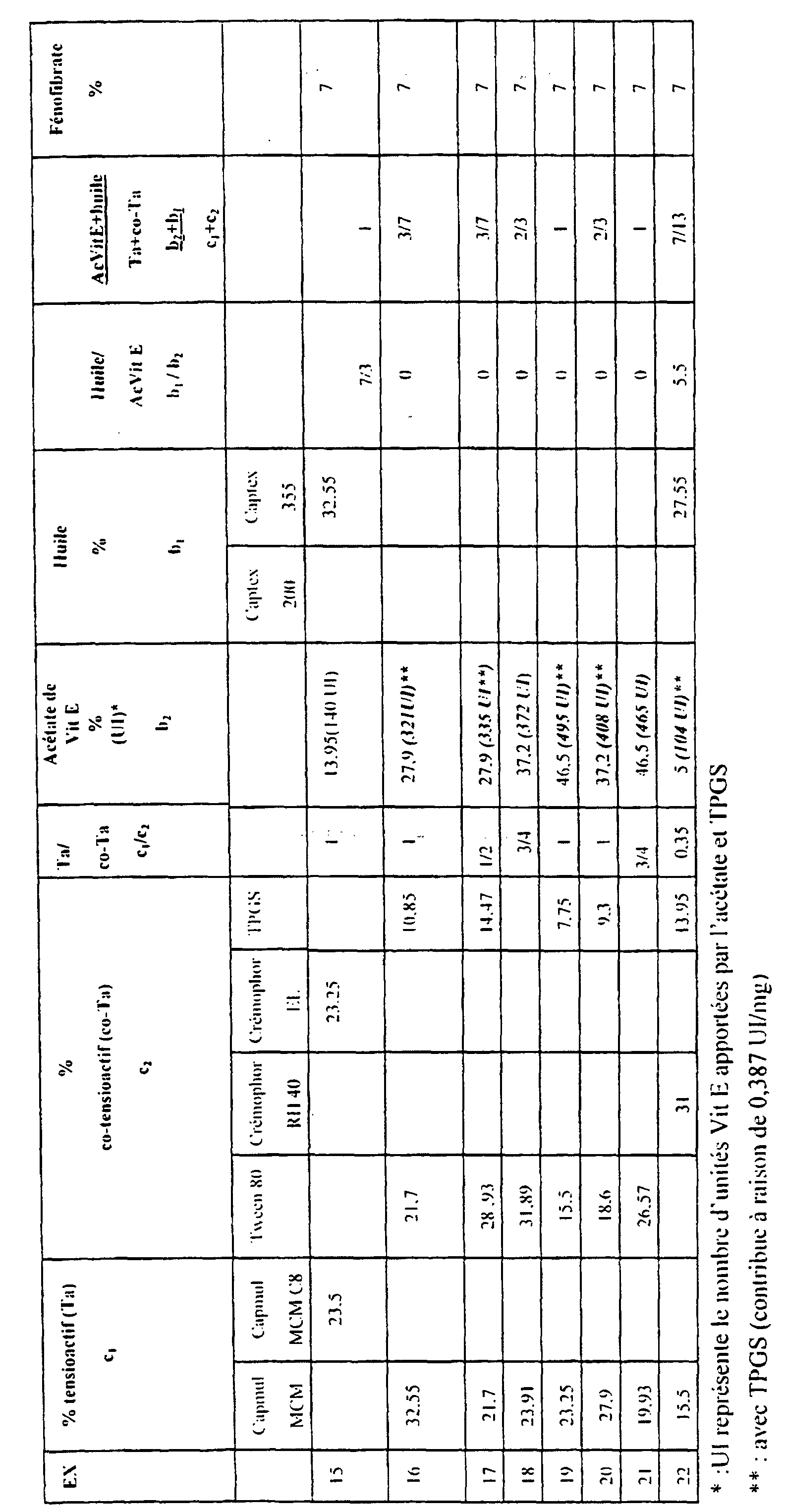

**TABLEAU II**

| **EX** | **AUC** | **TRM** | **Cmax** | **Tmax** |
|---|---|---|---|---|
| 7 | 8049 | 13.2 | 439 | 4 |
| 9 | 6902 | 13.4 | 361 | 12 |
| 10 | 6593 | 17.9 | 312 | 8 |
| 11 | 6298 | 11.8 | 393 | 4 |
| 12 | 6293 | 14.7 | 299 | 4 |
| 13 | 6169 | 13.8 | 325 | 8 |
| 14 | 6053 | 13.6 | 309 | 8 |
| 16 | 7166 | | | |
| 17 | 6872 | | | |
| 18 | 6695 | | | |
| 22 | 9820 | 13.8 | 454 | 4 |
| LIP | 3453 | 10.6 | 255 | 4 |
| Fen. Comp. | 5303 | 15.5 | 288 | 4 |

L'interprétation de ces résultats, après correction de la dose administrée, montre une amélioration très sensible de la biodisponibilité du fénofibrate lorsque celui-ci est formulé selon l'invention : l'aire sous courbe est nettement augmentée, ce qui témoigne d'une quantité plus importante de fénofibrate réellement absorbée.

De façon pratique, la composition pharmaceutique peut être conditionnée en doses contenant chacune 50 à 80 mg de fénofibrate sous forme de gélules scellées ou de capsules molles, adaptées à une posologie de l'ordre de 1 à 3 prises par jour incluant une quantité de vitamine E assurant une dose journalière minimum de 100 UI.

Dans le but de vérifier la bonne dispersion du préconcentré conforme à l'invention dans le milieu gastrique et l'obtention d'une microémulsion, on a effectué une mesure du diamètre moyen des gouttelettes formées dans la microémulsion après dilution du préconcentré.

La préparation de l'échantillon pour mesure de la taille a été conduite en effectuant une dilution du préconcentré, dans l'eau à 37 °C. Le diamètre moyen des gouttelettes est mesuré à l'aide d'un granulomètre Coulter N4 après un temps de stabilisation de 5 mn, sous un angle de 90°. Toutes les formulations réalisées conduisent à une microémulsion stable et limpide.

| Exemples | Taille des gouttelettes (nm) | Dilution |
|---|---|---|
| Exemple 7 | 17.4 ± 12.4 | 1/250^{ième} |
| Exemple 9 | 37.9 ± 12.4 | 1/250^{ième} |
| Exemple 12 | 32.6 ± 7 | 1/250^{ième} |
| Exemple 14 | 33.1 ± 11 | 1/250^{ième} |
| Exemple 16 | 49 ± 16 | 1/2000 |
| Exemple 17 | 58 | 1/2500 |
| Exemple 18 | 37 ± 9.5 | 1/1000 |
| Exemple 19 | 53 ± 9 | 1/2500 |
| Exemple 20 | 42.8 ± 12 | 1/2000 |
| Exemple 21 | 52 ± 10 | 1/2500 |
| Exemple 22 | 30.3 ± 10 | 1/500 |

La dilution du préconcentré dans l'eau ou dans une phase aqueuse conduit à une solution limpide, constituée d'une dispersion de gouttelettes dont le diamètre moyen est compris entre 18 et 60 nm; ces deux éléments étant la caractéristique d'une microémulsion. De plus une telle dispersion du principe actif favorise l'absorption et permet d'obtenir une biodisponibilité supérieure à celle obtenue précédemment avec les formulations connues.

## Revendications

1. Composition pharmaceutique pour l'administration par voie orale du fénofibrate sous forme d'un préconcentré de microémulsion huile-dans-eau comprenant :
- une phase lipophile ;
- un système émulsifiant comprenant un tensioactif lipophile et un co-tensioactif hydrophile ;
**caractérisée en ce qu'**elle comprend en outre de l'acétate de vitamine E en une quantité suffisante pour stabiliser ledit préconcentré, sans adjonction d'un composant hydrophile additionnel, autre que l'eau.

2. Composition pharmaceutique selon la revendication 1, **caractérisée en ce que** la phase lipophile précitée comprend une huile à base de glycérol ou de propylène glycol totalement estérifié par des acides gras saturés à chaîne moyenne.

3. Composition pharmaceutique selon la revendication 1 ou 2, **caractérisée en ce que** le tensioactif lipophile précité est à base de glycérol ou de propylèneglycol partiellement estérifié par des acides gras saturés à chaîne moyenne.

4. Composition pharmaceutique selon l'une des revendications 1 à 3, **caractérisée en ce qu'**elle contient, en quantités pondérales relatives :
a) 5 à 20 % de fénofibrate
b₁) 0 à 69 % d'huile
b₂) 5 à 76 % d'acétate de vitamine E
c₁) 3 à 69 % de tensioactif
c₂) 3 à 69 % de co-tensioactif.

5. Composition pharmaceutique selon l'une des revendications 1 à 4, **caractérisée en ce qu'**elle contient :
a) 6 à 9 % de fénofibrate
b₁) 0 à 40 % d'huile
b₂) 5 à 50 % d'acétate de vitamine E
c₁) 12 à 45 % de tensioactif
c₂) 12 à 45 % de co-tensioactif.

6. Composition pharmaceutique selon l'une des revendications 1 à 5, **caractérisé en ce que** le système émulsifiant comprend de la vitamine E TPGS (succinate de l' α-D-tocophérol et du polyéthylèneglycol 1000), de préférence en une quantité telle que la dose journalière de vitamine E administrée soit d'environ au moins 100 UI.

7. Composition pharmaceutique selon l'une des revendications 1 à 6, **caractérisée en ce que** :
• les composants b₁ et b₂ sont dans un rapport pondéral b₁/b₂ compris entre 0 et 15/1, de préférence entre 0 et 10/1;
• les composants c₁ et c₂ sont dans un rapport pondéral c₁/c₂ compris entre 1/80 et 4/1, de préférence entre 1/12 et 2/1; et
• les composants b₁,b₂, c₁ et c₂ sont tels que le rapport pondéral (b₁+b₂)/(c₁+c₂) est compris entre 1/15 et 5/1, de préférence entre 1/5 et 2/1.

8. Composition pharmaceutique selon l'une des revendications 1 à 7, **caractérisée en ce que** l'huile précitée est un triglycéride des acides caprylique et caprique.

9. Composition pharmaceutique selon l'une des revendications 1 à 8, **caractérisée en ce que** le tensioactif précité est un ester partiel du glycérol et des acides caprique et caprylique.

10. Composition pharmaceutique selon l'une des revendications 1 à 9, **caractérisée en ce que** le co-tensioactif est un polysorbate, de préférence le polysorbate 80.

11. Composition pharmaceutique selon l'une des revendications 1 à 9, **caractérisée en ce que** le co-tensioactif est un produit de réaction de l'huile de ricin, hydrogénée ou non hydrogénée, avec l'oxyde d'éthylène, tel que la HLB est supérieure à 12.

12. Composition pharmaceutique selon l'une des revendications 1 à 11, **caractérisée en ce qu'**elle se présente sous la forme de gélules scellées ou de capsules molles.

## Patentansprüche

1. Arzneimittel zur Verabreichung auf oralem Wege von Fenofibrat in Form eines Vorkonzentrats einer Mikroemulsion von Öl in Wasser, umfassend:
- eine lipophile Phase;
- ein Emulgatorsystem, umfassend ein lipophiles Tensid und ein hydrophiles Co-Tensid;
**dadurch gekennzeichnet, dass** sie außerdem Vitamin-E-Acetat in einer ausreichenden Menge, um das Vorkonzentrat, ohne Zusatz einer weiteren hydrophilen Komponente, außer Wasser, zu stabilisieren.

2. Arzneimittel gemäß Anspruch 1, **dadurch gekennzeichnet, dass** die vorgenannte lipophile Phase ein Öl auf der Basis von Glycerin oder Propylenglycol, das vollständig durch mittelkettige, gesättigte Fettsäuren verestert ist, umfasst.

3. Arzneimittel gemäß Anspruch 1 oder 2, **dadurch gekennzeichnet, dass** das vorgenannte lipophile Tensid auf der Basis von Glycerin oder Propylenglycol, das teilweise durch mittelkettige, gesättigte Fettsäuren verestert ist, ist.

4. Arzneimittel gemäß einem der Ansprüche 1 bis 3, **dadurch gekennzeichnet, dass** es, in relativen Gewichtsmengen, enthält:
a) 5 bis 20 % Fenofibrat
b₁) 0 bis 69 % Öl
b₂) 5 bis 76 % Vitamin-E-Acetat
c₁) 3 bis 69 % Tensid und
c₂) 3 bis 69 % Co-Tensid.

5. Arzneimittel gemäß einem der Ansprüche 1 bis 4, **dadurch gekennzeichnet, dass** es enthält:
a) 6 bis 9 % Fenofibrat
b₁) 0 bis 40 % Öl
b₂) 5 bis 50 % Vitamin-E-Acetat
c₁) 12 bis 45 % Tensid und
c₂) 12 bis 45 % Co-Tensid.

6. Arzneimittel gemäß einem der Ansprüche 1 bis 5, **dadurch gekennzeichnet, dass** das Emulgatorsystem Vitamin E TPGS (Succinat von α-D-Tocopherol und von Polyethylenglycol 1000), vorzugsweise in einer Menge, so dass die verabreichte Tagesdosis an Vitamin E etwa mindestens 100 IE beträgt, umfasst.

7. Arzneimittel gemäß Anspruch 4 oder 5, **dadurch gekennzeichnet, dass**:
- die Komponenten b₁ und b₂ in einem Gewichtsverhältnis b₁/b₂ zwischen 0 und 15/1, vorzugsweise zwischen 0 und 10/1, vorliegen;
- die Komponenten c₁ und c₂ in einem Gewichtsverhältnis c₁/c₂ zwischen 1/80 und 4/1, vorzugsweise zwischen 1/12 und 2/1, vorliegen; und
- die Komponenten b₁, b₂, c₁ und c₂ derart vorliegen, dass das Gewichtsverhältnis (b₁+b₂)/(c₁+c₂) zwischen 1/15 und 5/1, vorzugsweise zwischen 1/5 und 2/1, liegt.

8. Arzneimittel gemäß einem der Ansprüche 1 bis 7, **dadurch gekennzeichnet, dass** das vorgenannte Öl ein Triglycerid von Capryl- und Caprinsäuren ist.

9. Arzneimittel gemäß einem der Ansprüche 1 bis 8, **dadurch gekennzeichnet, dass** das vorgenannte Tensid ein partieller Ester von Glycerin und Caprin- und Caprylsäuren ist.

10. Arzneimittel gemäß einem der Ansprüche 1 bis 9, **dadurch gekennzeichnet, dass** das Co-Tensid ein Polysorbat, vorzugsweise Polysorbat 80 ist.

11. Arzneimittel gemäß einem der Ansprüche 1 bis 9, **dadurch gekennzeichnet, dass** das Co-Tensid ein Reaktionsprodukt von hydriertem oder nicht hydriertem Rizinusöl mit Ethylenoxid ist, so dass der HLB-Wert größer als 12 ist.

12. Arzneimittel gemäß einem der Ansprüche 1 bis 11, **dadurch gekennzeichnet, dass** es in Form von versiegelten Kapseln oder von Weichkapseln vorliegt.

## Claims

1. Pharmaceutical composition for the oral administration of fenofibrate in the form of a preconcentrate of an oil-in-water microemulsion, comprising:
- a lipophilic phase; and
- an emulsifying system comprising a lipophilic surfactant and a hydrophilic co-surfactant,
**characterized in that** it also comprises vitamin E acetate in a sufficient amount to stabilize said preconcentrate without the incorporation of an additional hydrophilic component other than water.

2. Pharmaceutical composition according to claim 1, **characterized in that** the above-mentioned lipophilic phase comprises an oil based on glycerol or propylene glycol totally esterified with medium-chain saturated fatty acids.

3. Pharmaceutical composition according to claim 1 or 2, **characterized in that** the above-mentioned lipophilic surfactant is based on glycerol or propylene glycol partially esterified with medium-chain saturated fatty acids.

4. Pharmaceutical composition according to one of claims 1 to 3,
**characterized in that** it contains the following in relative amounts by weight:
a) 5 to 20% of fenofibrate;
b₁) 0 to 69% of oil;
b₂) 5 to 76% of vitamin E acetate;
c₁) 3 to 69% of surfactant;
c₂) 3 to 69% of co-surfactant.

5. Pharmaceutical composition according to one of claims 1 to 4, **characterized in that** it contains:
a) 6 to 9% of fenofibrate;
b₁) 0 to 40% of oil;
b₂) 5 to 50% of vitamin E acetate;
c₁) 12 to 45% of surfactant;
c₂) 12 to 45% of co-surfactant.

6. Pharmaceutical composition according to one of claims 1 to 5, **characterized in that** the emulsifying system comprises vitamin E TPGS (α-D-tocopherol polyethylene glycol 1000 succinate), preferably in an amount such that the daily dose of vitamin E administered is at least about 100 IU.

7. Pharmaceutical composition according to one of claims 1 to 6, **characterized in that**:
• the components b₁ and b₂ are in a weight ratio b₁/b₂ of between 0 and 15/1, preferably of between 0 and 10/1;
• the components c₁ and c₂ are in a weight ratio c₁/c₂ of between 1/80 and 4/1, preferably of between 1/12 and 2/1; and
• the components b₁, b₂, c₁ and c₂ are such that the weight ratio (b₁ + b₂)/(c₁ + c₂) is between 1/15 and 5/ 1, preferably between 1/5 and 2/1.

8. Pharmaceutical composition according to one of claims 1 to 7, **characterized in that** the above-mentioned oil is a triglyceride of caprylic and capric acids.

9. Pharmaceutical composition according to one of claims 1 to 8, **characterized in that** the above-mentioned surfactant is a partial ester of glycerol and capric and caprylic acids.

10. Pharmaceutical composition according to one of claims 1 to 9, **characterized in that** the co-surfactant is a polysorbate, preferably polysorbate 80.

11. Pharmaceutical composition according to one of claims 1 to 9, **characterized in that** the co-surfactant is a reaction product of hydrogenated or non-hydrogenated castor oil with ethylene oxide such that the HLB is above 12.

12. Pharmaceutical composition according to one of claims 1 to 11, **characterized in that** it is presented in the form of sealed gelatin capsules or soft capsules.
